# EUROPÄISCHE PATENTANMELDUNG

(11) **EP 1 371 380 A1**
(43) Veröffentlichungstag der Anmeldung: **17.12.2003**
(21) Anmeldenummer: 03012854.0
(22) Anmeldetag: 06.06.2003
(51) Int. Cl.: A61M 1/00, A61B 17/32, A61B 17/22

(54) **Fettabsaugvorrichtung**

(30) Priorität: 13.06.2002 DE 10226250
(71) Anmelder: Möller Feinmechanik GmbH & Co. KG, 36043 Fulda (DE)
(72) Erfinder: Schröter, Werner, 36137 Grossenlüder (DE); Herget, Bernhard, 36115 Ehrenberg-Reulbach (DE)
(74) Vertreter: Schlagwein, Udo, Dipl.-Ing.

(57) **Zusammenfassung**

Zum Ansaugen von Körperfett werden Kanülen (2) benutzt, die in das Körperfettgewebe eingeführt werden. Diese sind üblicherweise mit einem Vibrationsantrieb (4) verbunden, um das Auftrennen des Körperfettgewebes zu erleichtern. Während einer Operation werden verschieden lange Kanülen (2) benutzt, so dass eine Auswechselmöglichkeit vorhanden sein muss.

Die Erfindung schlägt dazu eine leicht zu handhabende Kupplung (1) vor, die eine mechanische Verbindung mit dem Ausgangszapfen (3) des Vibrationsantriebes (4) herstellt.

Die Verbindung zu einer Saugvorrichtung zum Absaugen des Körperfettes wird hergestellt, indem ein Schlauch auf das Ende der Kanüle (2) aufgesteckt wird, die dazu seitlich abgewinkelt ist.

## Beschreibung

Die Erfindung bezieht sich auf eine Fettabsaugvorrichtung mit einem Vibrationsantrieb, an dessen Ausgangszapfen eine Kanüle lösbar befestigt ist, um eine oszillierende Längsbewegung der Kanüle zu bewirken, wobei im vorderen Abschnitt der Kanüle Öffnungen vorhanden sind und an dem hinteren Ende der Kanüle eine Absaugvorrichtung angeschlossen ist.

Eine derartige Vorrichtung ist zum Beispiel in dem Dokument EP 0 509 131 A1 beschrieben. Demnach ist das hintere Ende der Kanüle auf einen Ausgangszapfen eines Handgriffes klemmend aufgesteckt, der mittels eines Ultraschallerzeugers in Vibration versetzt wird. Durch den Handgriff und den Zapfen verläuft eine Längsbohrung, die mit der Absaugvorrichtung zum Absaugen von Körperfett verbunden ist. Mit der Steckverbindung wird nicht nur eine mechanische Verbindung zwischen der Kanüle und dem Handgriff bewirkt, sondern auch eine Leitungsverbindung zwischen der Kanüle und der Absaugvorrichtung hergestellt.

Der Ultraschallerzeuger ist lediglich in der Lage, leichte Vibrationen der Kanüle zu bewirken: Die Verbindung zwischen der Kanüle und dem Ausgangszapfen ist daher nur wenig mechanisch belastet, so dass eine Klemmverbindung als Kupplung ausreicht. Wenn ein Vibrationsantrieb eingesetzt werden soll, der höhere Frequenzen und größere Amplituden bewirkt, muss die Haltekraft der Kupplung zwischen der Kanüle verstärkt werden, damit sie sich während des Betriebes nicht löst, wobei schon ein leichtes Lösen dazu führen würde, dass Nebenluft in die Längsbohrung gelangt, was die Saugleistung senkt.

Außerdem muss auch eine verstärkte Kupplung leicht zu handhaben sein, da die Kanüle häufig während einer Operation gewechselt werden muss. Je nachdem, welche Körperbereiche in Bearbeitung sind, muss nämlich mit unterschiedlich langen Kanülen gearbeitet werden.

Die Erfindung beruht somit auf der Aufgabe, eine Fettabsaugvorrichtung zu schaffen, bei der eine sichere, sich während des Betriebes nicht lösende Verbindung zwischen der Kanüle und dem Vibrationsantrieb vorliegt, die darüber hinaus einfach zu handhaben ist.

Die Erfindung wird gelöst mit einer Fettabsaugvorrichtung gemäß dem Oberbegriff des Anspruchs 1 mit den weiteren Merkmalen, dass der hintere Abschnitt der Kanüle vor dem Anschluss für die Absaugvorrichtung gegenüber ihrem Hauptabschnitt seitlich abgewinkelt ist, wobei der Ausgangszapfen in der Verlängerung des Hauptabschnittes der Kanüle liegt, und dass zur lösbaren Befestigung mit dem Ausgangszapfen des Vibrationsantriebes eine Kupplung vorgesehen ist, dessen der Kanüle zugeordnetes Kanülenteil im Abwinkelbereich der Kanüle befestigt ist.

Mit einer solchen Lösung wird zunächst einmal erreicht, dass durch die getrennte Ausführung des Sauganschlusses und der mechanischen Befestigung der Kanüle am Vibrationsantrieb diese jeweils entsprechend den spezifischen Anforderungen realisiert werden können. Mit der Kupplung wird lediglich eine Verbindung geschaffen, mit der die Kanüle während des Betriebes sicher am Vibrationsantrieb gehalten ist. Da die Kupplung keine Leitungsverbindung realisiert, muss sie nicht so gestaltet sein, dass ein luftdichter Sauganschluss entsteht. Durch die Abwinkelung der Kanüle wird vielmehr erreicht, dass ein seitlicher Sauganschluss gelegt werden kann. Da die Abwinkelung nur wenige Grad beträgt, entsteht keine Knickstelle, in der eine Verstopfung auftreten könnte.

Da die Kupplung in der Verlängerung des Hauptabschnittes der Kanüle liegt, kann der Ausgangszapfen des Vibrationsantriebes und der Hauptabschnitt der Kanüle axial ausgerichtet werden, so dass die hin- und hergehende Bewegung unmittelbar und ohne lateralen Versatz auf die Kanüle übertragen wird. Dies hat den Vorteil, dass flatternde Schwingungen der Kanüle zur Seite vermieden werden, die wegen der versetzenden Krafteinleitung induziert würden.

Die Kupplung lässt sich dann besonders einfach realisieren, wenn es sich um eine Steckverbindung mit einem zylindrischen Steckerstift und einer zylindrischen Steckerbuchse handelt, deren Ausrichtung koaxial zum Ausgangszapfen bzw. zum Hauptabschnitt der Kanüle verläuft. Um die Kanüle mit dem Vibrationsantrieb zu verbinden, braucht lediglich die Steckerbuchse auf den Steckerstift aufgesteckt werden.

Um zu verhindern, dass sich die Kupplung löst, ist sie mit einem Drehverschluss, z. B. mit einem Bajonettverschluss, versehen. Dazu weist der Steckerstift mindestens zwei Nasen und die Steckerbuchse zwei Haken zum Hintergreifen der Nasen auf. Zum Zusammenstecken der Steckverbindung werden der Stecker und die Steckerbuchse so zueinander orientiert, dass die Haken an den Nasen vorbeigleiten können. Durch eine Drehung der Steckerbuchse auf dem Steckerstift werden die Haken hinter die Nasen gebracht, so dass ein Zurückziehen der Steckerbuchse vom Steckerstift und damit ein Lösen der Kupplung ausgeschlossen ist. Um ein Zurückdrehen zu verhindern, sind am Rücken der Nasen bzw. an zu den Haken führenden Laschen jeweils eine Rille bzw. ein damit korrespondierender Steg vorhanden, die einrastend ineinander greifen, wobei der damit verbundene Formschluss nur mit einem gewissen Kraftaufwand überwunden werden kann.

Um den einen Teil der Kupplung, nämlich den Kanülenteil mit der Kanüle zu verbinden, weist er einen Kanal auf, in dem der Abwinkelbereich der Kanüle passgenau liegt. Durch den abgewinkelten Verlauf der Kanüle ergibt sich außerdem ein Formschluss zwischen der Kanüle und dem Kanülenteil der Kupplung.

Der Kanal kann in einem Fortsatz am Kanülenteil fortgeführt werden, der bis zum hinteren Ende der Kanüle führt. Dort läuft der Fortsatz konisch aus und ist mit umlaufenden Rippen versehen, auf denen ein Schlauch der Absaugvorrichtung aufsteckbar ist.

Vorzugsweise besteht der Kanülenteil der Kupplung aus einem sterilisierbaren Kunststoff. Ein solches Kanülenteil lässt sich relativ leicht im Spritzgussverfahren realisieren.

Bei dem anderen Teil der Kupplung handelt es sich vorzugsweise um ein Metallteil, das auf dem Ausgangszapfen des Vibrationsantriebes aufgeschraubt ist.

Im Folgenden soll anhand eines Ausführungsbeispiels die Erfindung näher erläutert werden. Dazu zeigen
- Fig. 1: einen Längsschnitt durch eine erfindungsgemäße Kupplung mit einer Kanüle,
- Fig. 2-4: jeweils Querschnitte gemäß den Schnittlinien II - II, III - III bzw. IV - IV der Figur 1.

Eine Kupplung 1 verbindet eine Kanüle 2 mit dem Ausgangszapfen 3 eines Vibrationsantriebes 4, der, was hier nicht näher dargestellt ist, in einem griffartigen Gehäuse 5 untergebracht ist.

Der Vibrationsantrieb bewirkt im Wesentlichen eine hinund hergehende Bewegung des Ausgangszapfens 3 gemäß dem Doppelpfeil 6.

Die Kupplung 1 besteht aus einem ersten Teil, dem Kanülenteil 7, und einem zweiten Teil, dem Antriebsteil 8.

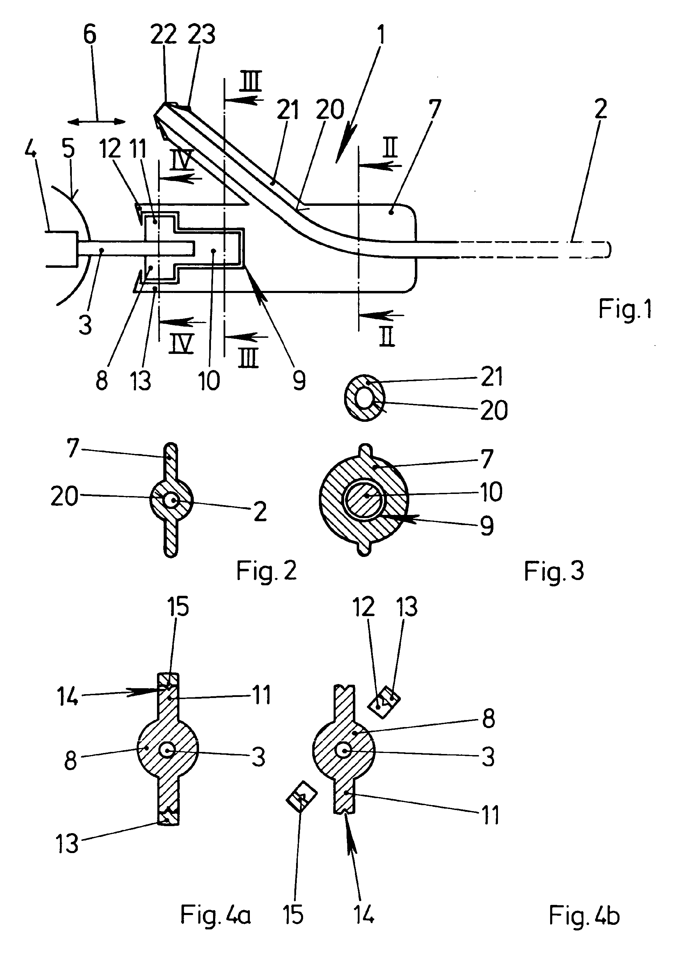

Im hinteren Bereich des Kanülenteiles 7 befindet sich eine Steckerbuchse 9 in Form einer zylinderförmigen Vertiefung, in der ein Stift 10 des Antriebsteiles 8 passgenau eingeführt werden kann. Deutlich ist dies an dem Schnitt der Figur 3 zu erkennen.

An dem Stift 10 befinden sich weiterhin zwei Nasen 11, die radial nach außen ragen und um 180° gegeneinander versetzt sind. Diese werden von Haken 12 an Laschen 13 hintergriffen, um ein Lösen der Steckverbindung zu verhindern. Figur 1 und die Schnittdarstellung der Figur 4a zeigen die geschlossene Position. Zum Schließen der Kupplung wird der Kanülenteil 7 zunächst gegenüber dem Antriebsteil 8 so ausgerichtet, dass sich die Situation der Figur 4b einstellt, in der Nasen 11 und Haken 12 gegeneinander versetzt sind. Die Steckerbuchse 9 kann nun auf den Steckerstift 10 aufgeschoben werden, wobei die Haken 12 an den Nasen 11 vorbei gleiten. Durch eine Drehung werden Haken 12 und Nasen 11 in eine Fluchtlinie gebracht, wodurch sich die in Figur 1 und Figur 4a dargestellte Situation einstellt. Um zu verhindern, dass sich der Drehverschluss löst, befinden sich an der Oberseite der Nasen Rillen 14 und an der Innenseite der Laschen 12 Stege 15, die in die Rillen 14 eingreifen, so dass ein Formschluss erzeugt wird, der nur mit einem gewissen Kraftaufwand wieder gelöst werden kann.

Im Kanülenteil 7 befindet sich ein Kanal 20, der an der Vorderseite des Kanülenteiles beginnt und sich zunächst noch axial erstreckt, dann aber seitlich abgewinkelt ist und in einen rohrförmigen Fortsatz 21 mit einer konischen Spitze 22 an seiner Spitze hineinläuft.

Entsprechend dem abgewinkelten Verlauf des Kanals 20 ist die Kanüle 2 abgewinkelt, so dass sie bis zum Ende des Fortsatzes 21 geführt wird. Dort wird ein hier nicht dargestellter Schlauch der Absaugvorrichtung aufgesteckt, wobei ein Abrutschen des Schlauches üblicherweise durch Rippen 23 an der Außenseite des rohrförmigen Fortsatzes verhindert wird.

Das Kupplungsteil ist aus Kunststoff gefertigt und wird im Spritzgussverfahren hergestellt, wobei in der Spritzgussform die gebogene Kanüle eingebracht wird, die damit den Kanal formt.

### Bezugszeichenliste

- 1: Kupplung
- 2: Kanüle
- 3: Ausgangszapfen
- 4: Vibrationsantrieb
- 5: Gehäuse

- 6: Doppelpfeil
- 7: Kanülenteil
- 8: Antriebsteil
- 9: Steckerbuchse
- 10: Steckerstift

- 11: Nasen
- 12: Haken
- 13: Laschen
- 14: Rillen
- 15: Stege

- 20: Kanal
- 21: Fortsatz
- 22: Spitze
- 23: Rippen

## Patentansprüche

1. Fettabsaugvorrichtung mit einem Vibrationsantrieb (4), an dessen Ausgangszapfen (3) eine Kanüle (2) lösbar befestigt ist, um eine oszillierende Längsbewegung der Kanüle (2) zu bewirken, wobei im vorderen Abschnitt der Kanüle (2) Öffnungen vorhanden sind und an dem hintere Ende der Kanüle (2) eine Absaugvorrichtung angeschlossen ist, **dadurch gekennzeichnet, dass** der hintere Abschnitt der Kanüle (2) vor dem Anschluss für die Absaugvorrichtung gegenüber ihrem Hauptabschnitt seitlich abgewinkelt ist, wobei der Ausgangszapfen (3) in der Verlängerung des Hauptabschnittes der Kanüle (2) liegt, und dass zur lösbaren Befestigung mit dem Ausgangszapfen (3) des Vibrationsantriebes (4) eine Kupplung (1) vorgesehen ist, dessen der Kanüle (1) zugeordnetes Kanülenteil (7) im Abwinkelbereich der Kanüle (1) befestigt ist.

2. Fettabsaugvorrichtung nach Anspruch 1, **dadurch gekennzeichnet, dass** es sich bei der Kupplung (1) um eine Steckverbindung mit einem zylindrischen Steckerstift (10) und einer zylindrischen Steckerbuchse (9) handelt, deren Ausrichtung koaxial zum Ausgangszapfen (3) bzw. zum Hauptabschnitt der Kanüle (2) verläuft.

3. Fettabsaugvorrichtung nach Anspruch 1 oder 2, **dadurch gekennzeichnet, dass** die Steckverbindung einen Drehverschluss aufweist.

4. Fettabsaugvorrichtung nach Anspruch 3, **dadurch gekennzeichnet, dass** am Steckerstift (10) mindestens zwei Nasen (11) und an der Steckerbuchse (9) zwei Haken (12) zum Hintergreifen der Nasen (11) vorhanden sind.

5. Fettabsaugvorrichtung nach Anspruch 4, **dadurch gekennzeichnet, dass** am Rücken der Nasen (11) und an zu den Haken (12) führenden Laschen (13) jeweils eine Rille (14) bzw. ein damit korrespondierender Steg (15) vorhanden ist.

6. Fettabsaugvorrichtung nach einem der vorhergehenden Ansprüche, **dadurch gekennzeichnet, dass** der Kanülenteil der Kupplung (1) einen Kanal (20) aufweist, in dem der Abwinkelbereich der Kanüle (1) passgenau liegt.

7. Fettabsaugvorrichtung nach Anspruch 6, **dadurch gekennzeichnet, dass** der Kanal (20) bis zum hinteren Ende eines rohrförmigen Fortsatzes (21) am Kanülenteil (7) verläuft, wobei der Fortsatz (21) dort konisch zuläuft und mit umlaufenden Rippen (23) versehen ist.

8. Fettabsaugvorrichtung nach Anspruch 7, **dadurch gekennzeichnet, dass** der Kanülenteil der Kupplung (1) aus einem sterilisierbaren Kunststoff besteht und an der Kanüle angespritzt ist.
